# FASCICULE DE BREVET EUROPEEN

(11) **EP 1 216 012 B1**
(45) Date de publication et mention de la délivrance du brevet: **30.08.2006**
(21) Numéro de dépôt: 00966237.0
(22) Date de dépôt: 29.09.2000
(51) Int. Cl.: A61F 5/00

(54) **IMPLANT GASTRIQUE REGLABLE**
EINSTELLBARES MAGENIMPLANTAT
ADJUSTABLE GASTRIC IMPLANT

(30) Priorité: 01.10.1999 FR 9912529
(43) Date de publication de la demande: 26.06.2002
(73) Titulaire: Medical Innovation Developpement, 69100 Villeurbanne (FR)
(72) Inventeur: LONGOBARDI, Bruno, F-69100 Villeurbanne (FR)
(74) Mandataire: Thibault, Jean-Marc
(86) Numéro de dépôt international: PCT/FR2000/002706
(87) Numéro de publication internationale: WO 2001/024742

(56) Documents cités:
- WO-A-94/27504
- DE-A- 19 751 733
- US-A- 5 074 868

## Description

### DOMAINE TECHNIQUE

L'invention a pour objet le domaine des implants gastriques qui sont adaptés autour de l'estomac du patient de manière à délimiter, en partie supérieure, une poche ou cavité à volume relativement restreint communiquant avec le reste de la poche stomacale par un chenal ou un conduit calibré par l'intermédiaire de l'implant.

Un tel domaine correspond à une méthode de lutte contre l'obésité laquelle a fait l'objet d'un certain nombre de propositions techniques visant à créer, par intervention et localement sur la poche stomacale, une striction locale réduisant la capacité d'absorption alimentaire.

### TECHNIQUE ANTERIEURE

Parmi les techniques connues, il convient de citer celle dite de dérivation consistant à isoler dans la poche stomacale une partie supérieure par agrafage ou analogue et à relier cette poche à la sortie de l'estomac par une dérivation. Cette technique est connue par le terme anglo-saxon *"gastric by-pass".*

Cette méthode consiste, en réalité, en une intervention lourde et pouvant être considérée comme irréversible sans que les résultats sur le contrôle du poids puissent être estimés complètement satisfaisants.

Une autre méthode proposée par la technique antérieure est connue sous la dénomination de *"gastroplastie verticale"* calibrée par un anneau élastique.

Une telle méthode implique de délimiter, à partir de la zone de raccordement entre l'oesophage et l'estomac, un chenal de section réduite par l'intermédiaire de plusieurs rangées d'agrafes et à la base duquel chenal un anneau élastique ouvert vient réaliser un effet de striction du chenal.

Une telle intervention est source de complications secondaires fréquentes qui impliquent un taux de réintervention élevé, alors que les statistiques font apparaître une tolérance alimentaire médiocre.

Une troisième technique consiste à disposer en position haute, sous hiatale, une bande gastrique réglable comportant, en relation avec la paroi extérieure de l'estomac, une cavité à volume variable pouvant être remplie d'un liquide par l'intermédiaire d'un boîtier de commande implanté en sous-cutané.

Cette technique connue sous la dénomination *"Adjustable Gastric Banding"* peut être considérée comme apportant les meilleurs résultats actuels pour différentes raisons.

La première est la possibilité d'intervention sous coelioscopie qui permet de bénéficier de conditions opératoires satisfaisantes et non traumatisantes. La seconde est la facilité d'ajuster l'effet de striction stomacale par l'intermédiaire du remplissage de la cavité remplie de liquide.

Pour mettre en oeuvre une telle technique, un certain nombre de propositions ont été exprimées et, parmi celles-ci, il peut être fait référence à l'enseignement de la demande EP 0 769 282 qui vise un dispositif pour la réduction de nourriture d'un patient, un tel dispositif comprenant une bande flexible mais non extensible, de largeur relativement faible, sur une des faces de laquelle est rapportée, généralement par collage, une enveloppe tubulaire réalisée en matière souple extensible.

L'enveloppe est raccordée par un tube à un boîtier pourvu d'une membrane auto-obturable qui peut être transpercée par une aiguille de seringue ou analogue, au moyen de laquelle une injection ou un prélèvement de liquide, tel que du sérum physiologique, peut être effectué pour contrôler le gonflement de l'enveloppe qui est chargée d'induire un effet de striction de la poche stomacale.

Des parties terminales sont prévues aux extrémités du dispositif, de manière à rendre possible une fermeture de la bande sous la forme d'une ceinture fermée par liaison des parties terminales, de telle manière que l'enveloppe contribue à former la surface périphérique interne de l'anneau.

Il peut être considéré qu'un tel dispositif permet de répondre à l'objectif d'implantation et de striction localisées de la poche stomacale mais les reculs d'implantation ont permis de mettre en évidence un certain nombre d'inconvénients tenant à la réalisation d'un tel dispositif.

Tout d'abord, les parties terminales, responsables de la liaison en boucle fermée de la bande, ne sont pas de nature à permettre la constitution d'un anneau de forme régulière s'approchant pratiquement d'une section circulaire théorique. Il s'ensuit que des zones de contraintes ponctuelles soumettent la paroi stomacale à des pressions localisées qui sont de nature à induire des phénomènes d'intolérance, voire, le cas échéant, d'être responsables de perforations locales. En outre, l'existence d'un anneau de striction dont la section interne n'est pas régulière n'est pas non plus favorable à la délimitation d'une section de passage réduite apte à l'établissement d'un transit basiquement approprié.

Il a, par ailleurs, été constaté que la bande allongée de nature souple mais non extensible possède, en raison de son mode de production notamment, des arêtes situées au niveau des bords longitudinaux qui offrent, en conséquence, une nature agressive susceptible de provoquer des traumatismes de la paroi stomacale.

Il a même été constaté que ces arêtes agressives pouvaient être responsables de perforations locales qui exigent, bien évidemment et très rapidement, une intervention de reprise.

Il a été constaté également que la forme d'exécution tenant à la réunion par collage de la bande et de l'enveloppe aboutit aussi, en raison de l'existence d'arêtes agressives, à un risque de perforation de l'enveloppe gonflée qui n'est plus alors à même d'assurer le calibrage du chenal tout en étant responsable d'un épanchement du liquide de gonflage même si ce dernier est généralement de nature physiologique.

Enfin, pour terminer, il convient de noter que la nature des moyens de liaison entre les parties terminales extrêmes confère à ces dernières, après liaison, une orientation divergente, à la manière des lames ouvertes d'une paire de ciseaux, et que, dans cet état, lesdites parties terminales sont alors souvent responsables de détérioration, d'irritation, voire de perforation de la paroi stomacale. Des implants gastriques comprenant les caractéristiques définies dans le préambule de la revendication 1 de la présente demande sont connus des documents US-5,074,868 et WO 94/27504.

La présente invention a pour objet des perfectionnements aux implants gastriques du type ci-dessus, ces perfectionnements étant réunis pour surmonter les inconvénients attachés aux solutions ou aux réalisations faisant intervenir, comme dit précédemment, une bande préfabriquée séparément et une enveloppe liée à cette bande par tout moyen d'attache, tel notamment que collage.

### EXPOSE DE L'INVENTION

Pour atteindre les objectifs ci-dessus, l'implant gastrique conforme à l'invention, est du type comprenant une bande en matière souple mais non extensible, associée à une enveloppe tubulaire en matière souple déformable, fermée à une extrémité et communiquant par l'autre avec un tube raccordé à un boîtier comportant une membrane auto-obturable, transperçable par une aiguille d'injection et/ou de prélèvement d'un fluide, pour commander à volonté le remplissage de l'enveloppe, ladite bande étant pourvue à ses extrémités de parties terminales complémentaires permettant de fermer la bande sous la forme d'un anneau dont la surface périphérique interne est occupée par l'enveloppe, ledit implant étant caractérisé en ce que :
- la bande est constituée par une pièce allongée rendue solidaire de l'intérieur de l'enveloppe et dont la largeur et l'épaisseur sont inférieures aux dimensions correspondantes de la section transversale droite et oblongue de l'enveloppe,
- la pièce possède des bords longitudinaux convexes,
- et des moyens complémentaire de liaison avec recouvrement sont prévus entre des parties terminales de la pièce qui font saillie hors de l'enveloppe.

Diverses autres caractéristiques ressortent de la description faite ci-dessous en référence aux dessins annexés qui montrent, à titre d'exemples non limitatifs, des formes de réalisation et de mise en oeuvre de l'objet de l'invention

### BREVE DESCRIPTION DES DESSINS

La **fig. 1** est une représentation schématique illustrant l'application de l'anneau gastrique selon l'invention.
La **fig. 2** est une vue en plan développée de l'implant gastrique conforme à l'invention.
La **fig. 3** est une élévation latérale prise selon la ligne **III-III** de la **fig. 2.**
La **fig. 4** est une vue transversale prise sensiblement selon la ligne **IV-IV** de la **fig. 2**.
La **fig. 5** est une vue de dessus montrant la mise en oeuvre de l'objet de l'invention.
La **fig. 6** est une coupe-élévation transversale d'une variante de réalisation.
Le **fig. 7** est une perspective d'une autre variante de réalisation.

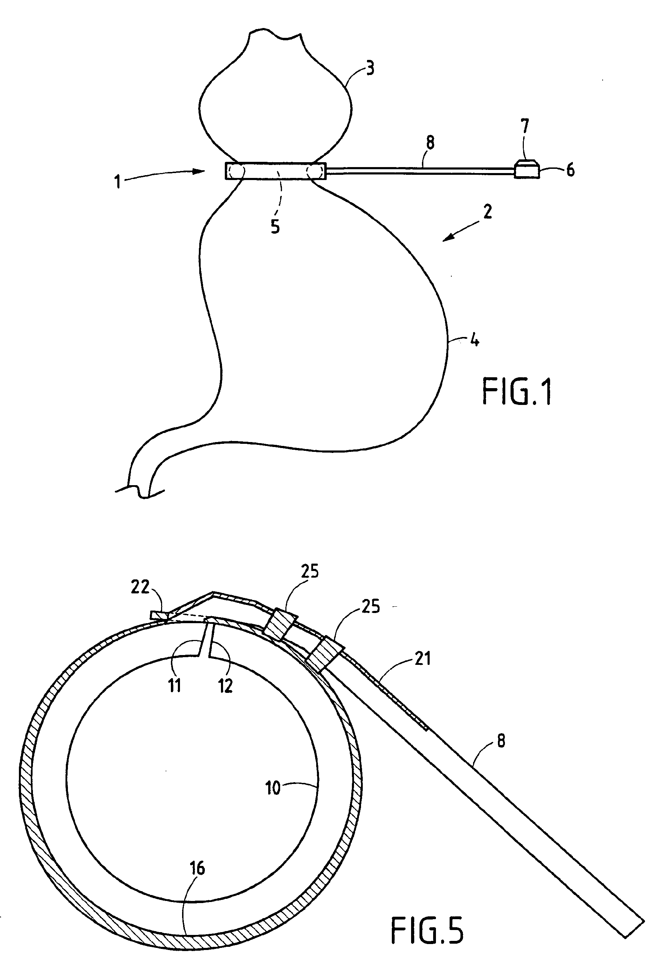

La **fig. 1** montre, de façon schématique, l'implantation d'un anneau gastrique désigné par la référence **1,** en position haute sous hiatale d'un estomac **2,** de manière à délimiter une poche gastrique artificielle supérieure **3** qui est en relation avec la poche gastrique inférieure **4** par l'intermédiaire d'un chenal de communication **5** dont la section de passage est contrôlée par l'implant gastrique **1.**

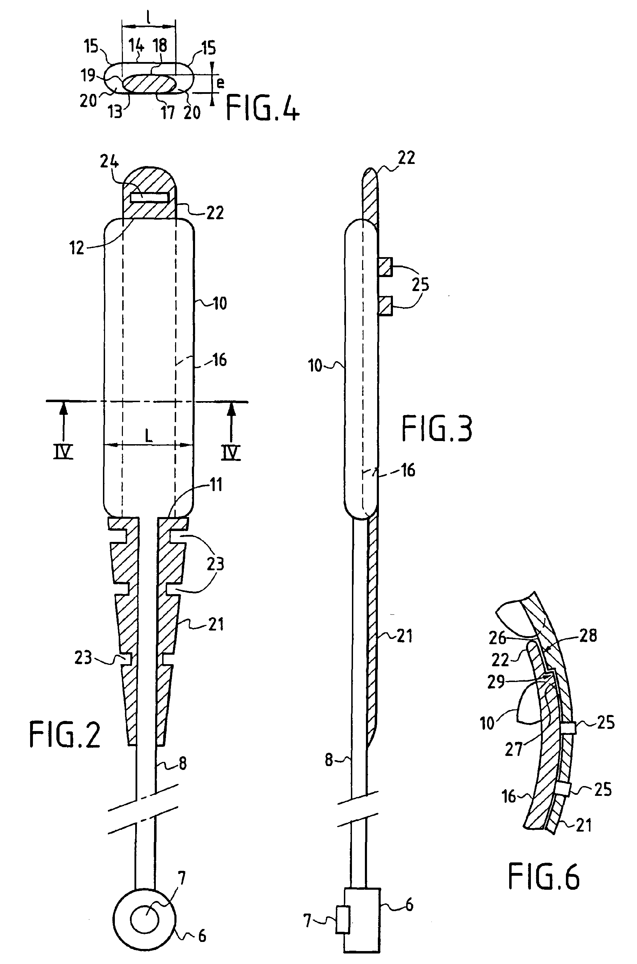

Selon une disposition connue, l'implant gastrique **1** est constitué sous la forme d'une ceinture, d'un anneau fermé, voire d'une frette, chargé de générer une striction dans la paroi stomacale par l'intermédiaire d'une enveloppe gonflable avec un liquide, tel que du sérum physiologique, qui est prélevé ou introduit dans ladite poche par l'intermédiaire d'un boîtier **6** possédant une membrane **7** auto-obturable qui peut être transpercée par l'intermédiaire de l'aiguille d'une seringue appropriée ou d'un moyen analogue. Le boîtier **6** est lié à l'enveloppe gonflable par l'intermédiaire d'un tube souple **8** dont la longueur et la souplesse sont choisis pour faciliter l'implantation sous-cutanée du boîtier **6.** L'implant gastrique **1** réalisé, conformément à l'invention, pour la fonction et l'application qui viennent d'être rappelées, se caractérise par une structure mise en évidence par les **fig. 2 à 4.**

### MEILLEURE MANIERE DE REALISER L'INVENTION

Selon ces figures, l'implant gastrique **1** comprend une poche ou une enveloppe **10** de forme généralement tubulaire, réalisée en une matière souple appropriée, déformable élastiquement. Le choix de cette matière relève de la compétence de l'homme du métier.

L'enveloppe tubulaire **10** est raccordée, par un première extrémité **11,** au tube **8** et se trouve fermée au droit de la seconde extrémité **12.**

L'enveloppe tubulaire **10** est réalisée de manière à présenter une section droite transversale oblongue qui est définie par deux côtés plans **13** et **14** qui sont raccordés entre eux par deux bords convexes **15** occupant les bords longitudinaux de l'enveloppe entre les extrémités **11** et **12.**

Selon une disposition constructive particulière de l'objet de l'invention, l'enveloppe **10** est associée à une pièce allongée **16** qui est constituée par une bande de matière souple mais non extensible qui présente, par ailleurs, la particularité d'être disposée à l'intérieur de l'enveloppe **10** pour être liée à la face interne de l'un des côtés plans, par exemple le côté **13** comme cela est illustré par la **fig. 4.** Le choix de la matière constitutive de l'enveloppe relève de la connaissance de l'homme du métier.

La pièce allongée **16** présente aussi la caractéristique de posséder, en section droite transversale, une forme oblongue dont la largeur **1** est inférieure à la largeur transversale L de l'enveloppe **10** et, de préférence, sensiblement égale à la longueur d'un côté plan **13** ou **14.** Selon une autre caractéristique, la pièce allongée **16** possède une épaisseur **e** inférieure à la distance séparant les côtés plans **13** et **14** et la section oblongue, qui lui est ainsi conférée, est définie par deux faces planes **17** et **18** qui sont raccordées par deux bords convexes **19.**

La pièce allongée **16** est liée par l'une de ses faces, par exemple **17,** à la surface intérieure de l'un des côtés plans, par exemple **13,** de l'enveloppe **10,** de manière que les cotés convexes **16** et les bords **19** délimitent entre eux, à partir du coté plan **13,** des sortes de demi-lunules **20** dont la fonction apparaît dans ce qui suit.

Conformément à l'invention, la structure décrite ci-dessus est obtenue d'une seule pièce, de façon monobloc pratiquement, par exemple par moulage.

La pièce allongée **16** présente, par ailleurs, une autre caractéristique constructive qui est celle de posséder une première partie terminale **21** qui s'étend hors de l'extrémité **11** de l'enveloppe **10,** en étant, pour partie au moins, accolée au tube **8,** de manière à assurer le renforcement de ce dernier. La pièce allongée **16** possède, par ailleurs, une seconde partie terminale **22** qui s'étend hors de l'extrémité **12** de l'enveloppe **10,** ladite extrémité étant obtenue, notamment dans le cas de fabrication quasi monobloc, par une liaison par collage sur la pièce allongée **16.**

Des moyens techniques sont prévus, entre les parties terminales **21** et **22,** de façon à permettre une liaison avec recouvrement entre elles, de telle manière que l'implant se présente alors sous la forme d'un anneau fermé de structure régulière, pratiquement circulaire et dont la paroi périphérique interne est occupée, comme cela sera vu ci-après, par l'enveloppe **10.**

De tels moyens de liaison comprennent, dans une forme de réalisation, des crans **23** qui sont ménagés dans les bords longitudinaux de la première partie terminale **21,** une fente **24** ménagée dans la seconde partie terminale **22** et au moins un et de préférence deux passants **25** qui sont formés en retrait de la partie terminale **22** et à proximité de cette dernière, tout en étant situés en retrait de l'extrémité **12** pour faire saillie transversalement à partir de la face extérieure du côté plan **13.** Dans l'exemple illustré, deux passants **25** sont prévus, chacun présentant la particularité de délimiter une sorte de pontet dont la largeur est sensiblement égale à celle de la partie terminale **21** prise à fond de crans **23.**

La structure de l'implant selon l'invention se caractérise par une conformation telle que l'implant gastrique, en raison de la disposition de la pièce allongée **16** à l'intérieur de l'enveloppe **10,** est dépourvu d'arêtes extérieures agressives, susceptibles de blesser la paroi stomacale. Par ailleurs, les conformations des côtés et bords convexes, laissant subsister les demi-lunules, permettent une déformation souple de l'enveloppe lors du gonflage sans qu'il en résulte de formation de plis ou d'arêtes encore susceptibles de blesser la paroi stomacale.

Enfin, comme il sera vu ci-après, les moyens de liaison avec recouvrement entre les parties terminales **21** et **22** maintiennent ces dernières dans un état s'inscrivant dans le profil de la paroi périmétrique de l'anneau fermé, ce qui permet aussi de réduire, voire supprimer, les risques de blessure de la paroi stomacale.

La mise en place de l'implant gastrique s'effectue de la façon suivante.

Dans l'état dégonflé de l'enveloppe **10,** les parties terminales **21** et **22** sont retournées l'une en direction de l'autre, de manière à former avec la bande un anneau, une ceinture, un bracelet ou une frette, de section sensiblement circulaire, tel qu'illustré par la **fig. 5.** Cette figure montre que l'enroulement de la bande constitutive de l'implant gastrique est effectué de telle manière que l'enveloppe gonflable **10** constitue la surface interne de l'anneau, alors que la pièce allongée **16** est orientée vers la périphérie extérieure.

Dans cette situation, les moyens de liaison avec recouvrement sont mis en oeuvre en procédant à l'engagement du tube **8** et de la première partie **21** à l'intérieur de la fente **24** de la seconde partie terminale **22,** de telle manière que la longueur excédentaire de la partie terminale **21** puisse être engagée ensuite à travers le ou les passants **25** au niveau desquels une liaison ferme, mais néanmoins amovible, intervient par l'engagement des passants **25** dans les crans **23.**

Selon une disposition préférée, il est avantageux, lorsque les passants sont au nombre de deux au moins, que leur écartement, pris selon la longueur de la bande gastrique, corresponde à l'écartement entre deux séries de crans **23.**

De la sorte, comme illustré par la **fig. 5,** les parties terminales **21** et **22** sont liées avec recouvrement l'une par rapport l'autre, ce qui permet, d'une part, de supprimer les parties terminales saillantes et divergentes telle que la technique antérieure les connaît et, d'autre part, de contribuer à la formation d'un anneau fermé dont la section s'approche le plus précisément d'une section de passage circulaire théorique favorable à l'implantation sur une paroi stomacale par nature relativement fragile.

Lorsque cette mise en place est intervenue, il suffit alors, par l'intermédiaire de l'aiguille d'une seringue engagée dans le boîtier **6** à travers la membrane **7,** d'injecter dans le tube **8,** et par conséquent dans l'enveloppe gonflable **10,** la quantité souhaitée de liquide, tel que du sérum physiologique, pour gonfler ladite enveloppe et, en conséquence, réduire la section de passage de l'anneau qui réalise un effet de striction sur la paroi stomacale pour calibrer le chenal de passage entre la poche artificielle sous hiatale **3** et la poche stomacale **4.**

Les moyens de liaison avec recouvrement entre les parties terminales **21** et **22** peuvent faire intervenir des variantes de réalisation telles que celle illustrée par la **fig. 6.** Dans une telle variante, la première partie terminale **21** possède, dans sa face **26** située en vis-à-vis de la face **27** de la partie terminale **22,** lorsque ces dernières sont en recouvrement, une conformation d'emboîtement **28** qui est complémentaire à une configuration de réception **29** présentée par la face **27.**

La conformation **28** est ménagée dans la zone de la partie terminale **21** proche de l'extrémité **11** et en amont de la série de crans **23** par rapport à cette extrémité, de manière à permettre la coopération desdits crans avec les passants **25** dans un état de coopération entre la conformation **28** et la configuration **29.**

Par les moyens décrits ci-dessus, les risques d'agressivité, de détérioration, voire de perforation dus au contact d'arêtes agressives avec la paroi stomacale sont en grande partie, voire totalement, supprimés. En outre, la fermeture de la bande constitutive de l'implant gastrique conduit à la formation d'un anneau de section régulière qui permet de contrôler précisément le calibre de passage du chenal de communication, entre la poche artificielle sous hiatale 3 et la poche stomacale **4.**

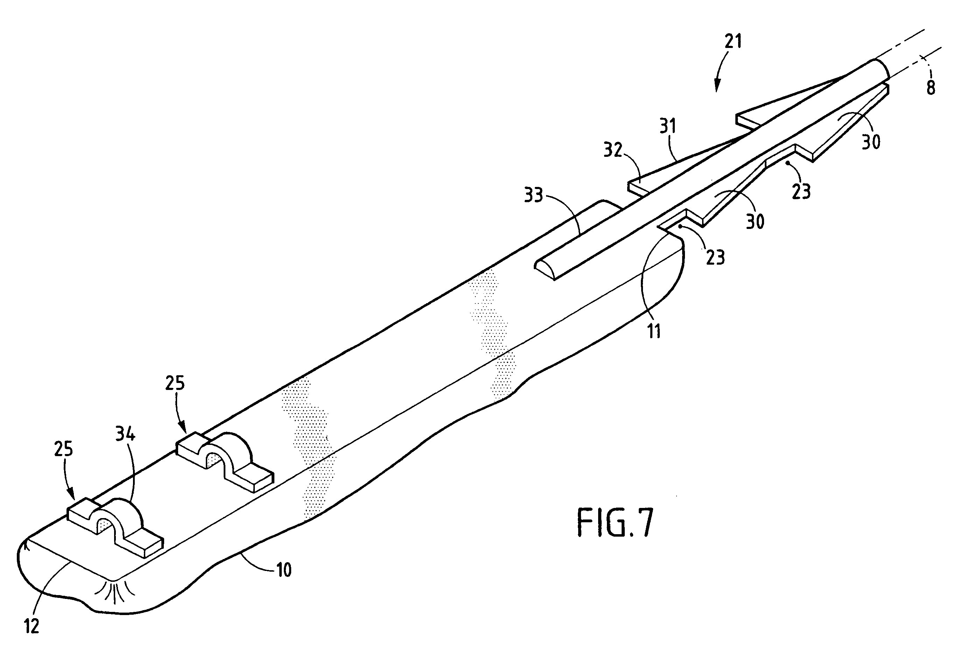

La **fig. 7** montre une autre variante de réalisation des moyens de liaison avec recouvrement. Dans cette variante, la partie terminale **21** présente une forme simple ou doublement lancéolée, chaque partie lancéolée, telle que **30,** possédant deux rampes inclinées d'engagement **31** aboutissant à deux épaulements **32** qui définissent, en quelque sorte, les crans **23.** Une telle conformation favorise l'engagement dans un ou plusieurs passants **25,** tout en assurant ensuite une liaison ferme apte à supporter une contrainte de traction par le gonflement de l'enveloppe **10.**

La partie **21** est aussi conformée pour admettre, par un conduit **33,** le raccordement ou le prolongement du tube **8** pour lequel une conformation complémentaire **34** est adoptée par les passants **25.** Une telle réalisation permet de supprimer la partie terminale **22.**

### APPLICATION INDUSTRIELLE

L'invention trouve une application avantageuse à la constitution d'implants gastriques réglables destinés à la lutte contre l'obésité.

L'invention n'est pas limitée aux exemples décrits et représentés car diverses modifications peuvent y être apportées sans sortir de son cadre.

## Revendications

1. Implant gastrique, du type comprenant une bande en matière souple mais non extensible, associée à une enveloppe tubulaire **(10)** en matière souple déformable, fermée à une extrémité **(12)** et communiquant par l'autre avec un tube **(8)** raccordé à un boîtier **(6)** comportant une membrane auto-obturable **(7)** transperçable par une aiguille d'injection et/ou de prélèvement d'un fluide pour commander, à volonté, le remplissage de l'enveloppe, ladite bande étant pourvue de moyens permettant de fermer la bande sous la forme d'un anneau dont la surface périphérique interne est occupée par l'enveloppe,
**caractérisé en ce que** :
- la bande est constituée par une pièce allongée **(16)** rendue solidaire de l'intérieur de l'enveloppe **(10)** qui est conformée pour présenter une section droite transversale oblongue définie par deux côtés plans **(13-14)** sensiblement parallèles entre eux et reliés par deux bords convexes **(15)** tandis que la pièce **(16)** présente une section droite transversale, également oblongue, dont la plus grande largeur est sensiblement égale à la longueur de l'un des côtés plans **(13-14).**
- la pièce possède des bords longitudinaux convexes **(19),**
- et des moyens complémentaires de liaison avec recouvrement (**23,24,25**) sont prévus entre les parties terminales de la bande.

2. Implant gastrique selon la revendication 1, **caractérisé en ce que** la pièce **(16),** l'enveloppe **(10),** les moyens de liaison avec recouvrement et le tube **(8)** sont réalisés de façon quasi monobloc.

3. Implant gastrique selon la revendication 1 ou 2, **caractérisé en ce que** la pièce **(16),** l'enveloppe **(10),** les moyens de liaison avec recouvrement et le tube **(8)** sont réalisés par moulage.

4. Implant selon la revendication 1, **caractérisé en ce que** la pièce **(16)** est définie par deux faces planes **(17** et **18)** et par deux bord arrondis **(19)** et **en ce que** ladite pièce est liée à l'intérieur de l'enveloppe par l'une de ses faces planes qui est coplanaire à la face interne de l'un des côtés plans de l'enveloppe.

5. Implant selon la revendication 1 ou 4, **caractérisé en ce que** la pièce **(16)** comporte deux parties terminales **(21-22)** s'étendant hors de l'enveloppe et dont l'une, dite la première, est raccordée au tube **(8)** et **en ce que** les moyens de liaison avec recouvrement entre ces parties terminales comprennent au moins une série de crans **(23)** présentés par ladite première partie et une série de passants **(25)** prévus en retrait de l'extrémité de la bande opposée à la première partie terminale.

6. Implant selon la revendication 5, **caractérisé en ce que** les moyens de liaison avec recouvrement comprennent, sur l'une des faces de la première partie terminale, une conformation **(28)** d'emboîtement ménagée à proximité de la zone de raccordement entre ladite première partie terminale et l'enveloppe et, sur la face en vis-à-vis de la seconde partie terminale **(22),** une configuration complémentaire **(29).**

7. Implant selon la revendication 1, **caractérisé en ce que** les moyens de liaison avec recouvrement comprennent uniquement une partie terminale **(21)** pourvue d'au moins une série de crans **(23)** et s'étendant à partir de l'une des extrémités fermée **(11)** de la bande et des passants **(25)** prévus en retrait de la seconde extrémité fermée **(12)** de ladite bande.

8. Implant selon la revendication 7, **caractérisé en ce que** la partie terminale **(21)** présente une conformation doublement lancéolée, délimite un conduit **(33)** correspondant au tube **(8)** et **en ce que** les passants **(25)** adoptent une conformation **(34)** complémentaire audit conduit.

## Claims

1. A gastric implant of the type comprising a strap of material that is flexible but not stretchable associated with a tubular bag (10) of deformable flexible material that is closed at one end (12) and that communicates via its other end with a tube (8) connected to a box (6) having a self-sealing membrane (7) that can be pierced by a needle for injecting and/or removing a fluid so as to control, at will, the extent to which the bag is filled, said strap being provided with means enabling it to be looped in the form of a closed band whose inner peripheral surface is occupied by the bag,
the implant being **characterized in that**:
. the strap is constituted by an elongate piece (16) secured to the inside of the bag (10) which is shaped to present a right cross-section that is oblong, being defined by two substantially parallel plane sides (13-14) interconnected by two convex edges (15), while the piece (16) has a right cross-section that is likewise oblong, with the greatest width thereof being substantially equal to the width of one of the plane sides (13-14);
. the piece possesses convex longitudinal edges (19); and
. complementary overlapping connection means (23, 24, 25) are provided between the end portions of the strap.

2. A gastric implant according to claim 1, **characterized in that** the piece (16), the bag (10), the overlapping connection means, and the tube (8) are made in substantially one-piece manner.

3. A gastric implant according to claim 1 or claim 2, **characterized in that** the piece (16), the bag (10), the overlapping connection means, and the tube (8) are made by molding.

4. An implant according to claim 1, **characterized in that** the piece (16) is defined by two plane faces (17 and 18) and by two rounded edges (19) and **in that** said piece is connected to the inside of the bag via one of its plane faces which is coplanar with the inside face of one of the plane sides of the bag.

5. An implant according to claim 1 or claim 4, **characterized in that** the piece (16) has two end portions (21-22) extending beyond the bag, with a first one of them being connected to the tube (8), and **in that** the overlapping connection means between said end portions comprise at least one series of notches (23) presented by said first portion and a series of D-loops (25) provided set back from the end of the strap opposite to the first end portion.

6. An implant according to claim 5, **characterized in that** the overlapping connection means comprise, on one of the faces of the first end portion, an engagement shape (28) formed close to the connection zone between the first end portion and the bag, and on the facing face of the second end portion (22), a complementary shape (29).

7. An implant according to claim 1, **characterized in that** the overlapping connection means comprise solely an end portion (21) provided with at least one series of notches (23) and extending from one of the closed ends (11) of the strap, together with D-loops (25) provided set back from the second closed end (12) of said strap.

8. An implant according to claim 7, **characterized in that** the end portion (21) presents a shape with two spearhead pieces, defining a duct (33) corresponding to the tube (8), and **in that** the D-loops (25) are of a shape (34) that is complementary to said duct.

## Patentansprüche

1. Magenimplantat der Art, die einen Streifen aus flexiblem aber nicht dehnbarem Material umfaßt, und verknüpft mit einer röhrenförmigen Hülle (10) aus flexiblem verformbarem Material, die an einem Ende (12) geschlossen ist und die durch das andere Ende mit einer Röhre (8) kommuniziert, die mit einem Gehäuse (6) verbunden ist, das eine selbstverschließbare Membran (7) umfaßt, die von einer Einspritz- und /oder Entnahmenadel eines Fluids durchstechbar ist, um das Füllen der Hülle wie gewünscht zu steuern, wobei der Streifen mit Mitteln versehen ist, die das Schließen des Streifens in der Form eines Rings erlauben, dessen innere Umfangsfläche von der Hülle besetzt ist,
**dadurch gekennzeichnet, daß**:
- der Streifen aus einem gestreckten Teil (16) besteht, das am Innen der Hülle (10) befestigt ist, die angepaßt ist, einen geraden länglichen Querschnitt aufzuweisen, der von zwei ebenen, im wesentlichen zueinander parallelen Seiten (13-14), die miteinander durch zwei konvexe Kanten (15) verbunden sind, definiert ist, während das Teil (16) einen geraden Querschnitt aufweist, der ebenso länglich ist und dessen größere Breite im wesentlichen gleich der Länge einer der ebenen Seiten (13-14) ist,
- das Teil zwei konvexe Längskanten (19) umfaßt,
- und komplementäre Verbindungsmittel mit Überdeckung (23, 24, 25) zwischen den Endteilen des Streifens vorgesehen sind.

2. Magenimplantat nach Anspruch 1, **dadurch gekennzeichnet, daß** das Teil (16), die Hülle (10) die Verbindungsmittel mit Überdeckung und die Röhre (8) quasi einteilig hergestellt sind.

3. Magenimplantat nach einem der Ansprüche 1 oder 2, **dadurch gekennzeichnet, daß** das Teil (16), die Hülle (10), die Verbindungsmittel mit Überdeckung und die Röhre (8) durch Formung ausgeführt sind.

4. Implantat nach Anspruch 1, **dadurch gekennzeichnet, daß** das Teil (16) von zwei ebenen Seiten (17 und 18) und zwei gerundeten Kanten (19) definiert ist und, daß das Teil mit dem Innen der Hülle durch eine seiner ebenen Seiten verbunden ist, die zur inneren Seite einer der ebenen Seiten der Hülle koplanar ist.

5. Implantat nach einem der Ansprüche 1 oder 4, **dadurch gekennzeichnet, daß** das Teil (16) zwei Endteile (21-22) umfaßt, die sich außerhalb der Hülle erstrecken und von denen der eine, der der erster Teil genannt wird, mit der Röhre (8) verbunden ist und, daß die Verbindungsmittel mit Überdeckung zwischen diesen Endteilen mindestens eine Reihe von Einkerbungen (23), die von dem ersten Teil aufgewiesen sind, und eine Reihe von Schlaufen (25) umfassen, die zurückversetzt vom Ende des Streifens, das gegenüber dem ersten Endteil liegt, vorgesehen sind.

6. Implantat nach Anspruch 5, **dadurch gekennzeichnet, daß** die Verbindungsmittel mit Überdeckung an einer der Seiten des ersten Endteils eine Verschachtelungskonfiguration (28), die in der Nähe des Verbindungsbereiches zwischen dem ersten Endteil und der Hülle angeordnet ist, und eine komplementäre Konfiguration (29) an der Seite, die gegenüber dem zweiten Endteil (22) liegt, umfassen.

7. Implantat nach Anspruch 1, **dadurch gekennzeichnet, daß** die Verbindungsmittel mit Überdeckung nur einen Endteil (21) umfassen, der mindestens mit einer Reihe von Einkerbungen (23) versehen ist und der sich ab einem der geschlossenen Enden (11) des Streifens und der Schlaufen (25) erstreckt, die aufwärts zum zweiten geschlossenen Ende (12) des Streifens vorgesehen sind.

8. Implantat nach Anspruch 7, **dadurch gekennzeichnet, daß** der Endteil (21) eine doppelt lanzettenförmige Gestaltung aufweist, eine Leitung (33), die der Röhre (8) entspricht, begrenzt, und, daß die Schlaufen (25) eine Konformation (34) aufweisen, die zur Leitung komplementär ist.
